(19) Europäisches Patentamt / European Patent Office / Office européen des brevets

(11) **EP 2 881 140 B1**

(12) **FASCICULE DE BREVET EUROPEEN**

(45) Date de publication et mention
de la délivrance du brevet:
**16.03.2016 Bulletin 2016/11**

(51) Int Cl.:
*A61N 1/362* *(2006.01)*    *A61N 1/368* *(2006.01)*

(21) Numéro de dépôt: **14186895.0**

(22) Date de dépôt: **29.09.2014**

(54) **Dispositif médical implantable actif à stimulation double chambre pour le traitement de l'insuffisance cardiaque à fraction d'éjection préservée HFpEF**

Implantierbare aktive medizinische Vorrichtung zur Doppelkammer-Stimulation für die Behandlung von Herzinsuffizienz mit erhaltener Auswurffraktion (HFpEF)

Active implantable medical device with dual chamber stimulation for treating cardiac insufficiency with HFpEF preserved ejection fraction

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.12.2013 FR 1362123**

(43) Date de publication de la demande:
**10.06.2015 Bulletin 2015/24**

(73) Titulaire: **Sorin CRM SAS
92140 Clamart Cedex (FR)**

(72) Inventeurs:
• **Amblard, Amel**
  **92330 Sceaux (FR)**
• **Limousin, Marcel**
  **75014 Paris (FR)**

(74) Mandataire: **Dupuis-Latour, Dominique
Bardehle Pagenberg
10, boulevard Haussmann
75009 Paris (FR)**

(56) Documents cités:
**EP-A1- 2 471 575    WO-A2-2010/071849
US-B1- 8 583 234**

• **EICHER JEAN CHRISTOPHE ET AL: "Permanent left atrial pacing therapy may improve symptoms in heart failure patients with preserved ejection fraction and atrial dyssynchrony: a pilot study prior to a national clinical research programme",** EUROPEAN JOURNAL OF HEART FAILURE, ELSEVIER, AMSTERDAM, NL, vol. 15, no. 1, 1 janvier 2013 (2013-01-01), pages 85-93, XP009177280, ISSN: 1388-9842, DOI: 10.1093/EURJHF/HFS150

EP 2 881 140 B1

**Description**

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle concerne plus particulièrement les dispositifs destinés à traiter l'insuffisance cardiaque (HF, *Heart Failure*), en alternative ou en complément au traitement des troubles du rythme cardiaque.

Cette thérapie vise à resynchroniser la contraction des cavités cardiaques (oreillette et ventricule, et les deux ventricules entre eux) de manière à améliorer l'état du patient par optimisation des différentes phases du cycle hémodynamique, ce cycle comprenant : pré-éjection, contraction isovolumique, éjection systolique, relaxation isovolumique, et enfin remplissage de la cavité.

La plupart de ces dispositifs mettent en oeuvre une technique dite "CRT" (*Cardiac Resynchronization Therapy*) ou "BVP" (*Bi-Ventricular Pacing*) consistant à délivrer en tant que de besoin des impulsions électriques permettant d'assurer une stimulation conjointe et permanente des deux ventricules, gauche et droit, afin de resynchroniser ces derniers.

Cette technique de resynchronisation biventriculaire ne s'adresse toutefois qu'à l'une des formes d'insuffisance cardiaque, dite "insuffisance systolique". Dans cette forme de la maladie, le muscle cardiaque est dans l'incapacité de fournir l'effort nécessaire pour assurer un débit cardiaque suffisant, et le patient présente des signes de dilatation entrainant un retard de dépolarisation du ventricule gauche. La stimulation biventriculaire CRT permet alors de resynchroniser les ventricules et de rendre plus homogène la contraction cardiaque.

L'autre forme d'insuffisance cardiaque, dite "insuffisance diastolique" ou "à fraction d'éjection préservée" (HFpEF, *Heart Failure with preserved Ejection Fraction*) ne présente pas de caractère de désynchronisation des ventricules, mais provient d'un défaut de remplissage du ventricule gauche.

Une stimulation CRT biventriculaire sera donc sans effet dans ce cas de figure.

Or cette pathologie touche près de 40 % des insuffisants cardiaques, et il n'existe pas de traitement efficace connu pour y remédier.

Cette forme de pathologie peut être chez certains patients la conséquence d'un trouble de conduction au niveau des oreillettes (bloc interauriculaire), qui induit un retard de la dépolarisation, et donc de la contraction, de l'oreillette gauche (OG) par rapport à l'oreillette droite (OD). En revanche, comme les voies de conduction atrioventriculaires ne sont pas altérées, la dépolarisation et la contraction des deux ventricules droit (VD) et gauche (VG) surviennent dans un délai normal, sans désynchronisation VD-VG. C'est entre la contraction de l'oreillette gauche et celle du ventricule gauche que le bloc interauriculaire OD-OG engendre un mauvais séquencement OG-VG : le retard de la contraction de l'oreillette gauche fait que celle-ci se contracte pratiquement en même temps que le ventricule gauche, et donc ne peut pas remplir correctement sa fonction et contribuer au remplissage actif du ventricule gauche.

Pour traiter cette pathologie d'insuffisance cardiaque à fraction d'éjection préservée, il a été proposé une technique *d'overdriving* auriculaire, consistant à stimuler l'oreillette gauche à une fréquence légèrement supérieure à la fréquence spontanée du rythme sinusal (donc du rythme de l'oreillette droite), de manière à provoquer systématiquement une dépolarisation prématurée de l'oreillette gauche et rétablir de ce fait une séquence OG-VG quasiment normale.

Plus précisément, dans cette technique connue, le dispositif évalue régulièrement le rythme spontané du patient et applique une séquence d'impulsions de stimulation à un rythme légèrement plus rapide, arbitrairement programmé pour provoquer une prématurité de l'ordre de 50 à 100 ms par rapport à un intervalle de couplage auriculaire correspondant au rythme sinusal spontané. Après plusieurs cycles à ce rythme accéléré, la fréquence est ralentie progressivement jusqu'à réapparition de l'activité spontanée, puis le processus *d'overdriving* est réitéré de la même façon et ainsi de suite.

La fréquence de stimulation appliquée varie donc en continu entre des valeurs où elle est trop rapide (période *d'overdriving*) ou trop lente (période de réapparition du rythme spontané, avec désynchronisation OD-VD), sans véritable contrôle de l'efficacité d'un éventuel retour à une synchronisation convenable des cavités gauches.

On notera également que ce mode de stimulation peut interférer sur le remplissage des cavités droites : en effet, du fait de la stimulation prématurée de l'oreillette gauche, la synchronisation OD-VD est significativement modifiée, d'une manière qui peut être incompatible avec un remplissage satisfaisant du ventricule droit (on explicitera ces aspects dans la description détaillée). La thérapie d'amélioration du remplissage du ventricule gauche est donc susceptible d'induire des effets délétères sur le remplissage du ventricule droit, donc du côté qui n'était pas affecté par la pathologie que l'on cherche à traiter.

Une autre technique est décrite dans le EP 2 471 575 A1 (Sorin CRM SAS) qui, notamment pour pallier ces inconvénients, met en oeuvre un capteur de recueil d'un signal d'accélération endocardiaque (EA). Le signal EA est analysé de manière à y détecter la présence d'une composante spécifique reflétant la contraction auriculaire (composante EA4) et à identifier l'instant d'apparition de cette composante. Si la composante EA4 est présente, ceci signifie que le séquencement des contractions auriculaires est correct, car dans le cas contraire (contractions auriculaires gauches trop tardives), la composante EA4 se trouverait noyée dans la composante du signal EA correspondant à la contraction ventriculaire immédiatement consécutive (composante EA1). L'intervalle de stimulation interauriculaire (intervalle AA) est alors ajusté dynamiquement en fonction du résultat de cette analyse.

Cette technique impose cependant de disposer d'une sonde implantée pourvue d'un capteur d'accélération endocardiaque, ainsi que d'un générateur susceptible de traiter les signaux EA délivrés par un tel capteur. Une technique comparable est décrite par le US 8 583 234 B1, qui prévoit en outre, après un premier cycle cardiaque sans stimulation auriculaire gauche, de délivrer une impulsion *d'overdriving* au cours d'un deuxième cycle cardiaque immédiatement consécutif.

L'un des buts de l'invention est de proposer une nouvelle technique de traitement de l'insuffisance cardiaque à fraction d'éjection préservée chez les patients souffrant d'un délai interauriculaire mécanique, qui pallie les inconvénients des méthodes proposées jusqu'à présent, et qui ne nécessite pas de recourir à des moyens de recueil et d'analyse d'un signal EA. Un autre but de l'invention est de proposer une telle technique qui assure un rétablissement de la fonction diastolique de manière à la fois simple (en termes de moyens mis en oeuvre) et très réactive (efficacité obtenue cycle à cycle) en permettant au patient de retrouver un séquencement OG-VG satisfaisant (séquencement de la contraction de l'oreillette gauche par rapport à celle du ventricule gauche), de manière que l'oreillette puisse remplir correctement sa fonction d'achèvement du remplissage du ventricule gauche.

Un autre but encore de l'invention est d'aboutir à un tel résultat au moyen d'un dispositif double chambre conventionnel (avec détection/stimulation auriculaire gauche et détection ventriculaire, mais sans détection des signaux de dépolarisation de l'oreillette droite), et ceci par une simple re-programmation des circuits de pilotage de cet appareil, donc sans modification *hardware*.

Plus précisément, l'invention propose un dispositif comprenant, de manière en elle-même connue, notamment d'après le US 8 583 234 B1 précité : des moyens de recueil de dépolarisations ventriculaires ; des moyens de recueil de dépolarisations auriculaires gauches ; des moyens de délivrance d'impulsions de stimulation auriculaire gauche ; et des moyens de contrôle de stimulation auriculaire, aptes à délivrer sélectivement lesdites impulsions de stimulation auriculaires gauches de manière prématurée, par application d'un délai interauriculaire gauche abrégé, plus court que l'intervalle de couplage du rythme sinusal. Après un premier cycle cardiaque sans stimulation auriculaire gauche, les moyens de contrôle de stimulation auriculaire délivrent une impulsion de stimulation auriculaire gauche prématurée au cours d'un deuxième cycle cardiaque immédiatement consécutif, avec application d'un délai interauriculaire gauche abrégé, plus court que l'intervalle de couplage du rythme sinusal. De façon caractéristique de l'invention, les moyens de contrôle de stimulation auriculaire opèrent de manière à :

- délivrer une impulsion de stimulation auriculaire gauche non prématurée au cours d'un troisième cycle cardiaque immédiatement consécutif audit deuxième cycle cardiaque, avec application d'un délai interauriculaire gauche correspondant à l'intervalle de couplage du rythme sinusal ; et

- évaluer l'intervalle atrioventriculaire séparant la dépolarisation auriculaire gauche de la dépolarisation ventriculaire auxdits deuxième et troisième cycles cardiaques, ou le délai interventriculaire séparant deux dépolarisations ventriculaires successives, comparer l'intervalle atrioventriculaire ainsi évalué à l'intervalle atrioventriculaire en rythme sinusal ou le délai interventriculaire ainsi évalué au délai interventriculaire en rythme sinusal, et modifier, ou non, au moins un paramètre des moyens de contrôle de stimulation auriculaire, tel que le délai interauriculaire gauche abrégé, en fonction du résultat de la comparaison.

Selon diverses caractéristiques subsidiaires avantageuses :

- si l'intervalle atrioventriculaire augmente au-delà d'une valeur-cible donnée, ou si le délai interventriculaire est inférieur à l'intervalle de couplage du rythme sinusal, il est prévu de : réduire d'un pas de variation ledit délai interauriculaire gauche abrégé ; puis délivrer une impulsion de stimulation auriculaire gauche prématurée au cours d'un cycle cardiaque consécutif, avec application dudit délai interauriculaire gauche abrégé réduit d'un pas ;

- inversement, si l'intervalle atrioventriculaire n'augmente pas au-delà de ladite valeur-cible et que le délai interventriculaire demeure voisin de l'intervalle de couplage du rythme sinusal, il est prévu de : augmenter d'un pas de variation ledit délai interauriculaire gauche abrégé ; puis délivrer une impulsion de stimulation auriculaire gauche prématurée au cours d'un cycle cardiaque consécutif, avec application dudit délai interauriculaire gauche abrégé accru d'un pas ;

- le dispositif comprend en outre des moyens aptes, sur détection d'un évènement prédéterminé, à : inhiber la délivrance de toute impulsion de stimulation auriculaire gauche au cours d'au moins un cycle cardiaque ; mesurer sur au moins un cycle cardiaque l'intervalle de couplage ventriculaire ; et mémoriser cet intervalle de couplage ventriculaire en tant qu'intervalle de couplage du rythme sinusal pour la détermination desdits délais interauriculaire gauche abrégé et interauriculaire gauche correspondant à l'intervalle de couplage du rythme sinusal, au cours des cycles ultérieurs ;

- cet évènement prédéterminé est l'expiration d'un intervalle de temps fixe prédéterminé, ou bien la détection du dépassement d'un seuil prédéterminé par la variation du délai atrioventriculaire et/ou de l'intervalle de couplage auriculaire et/ou du délai interventriculaire ;
- le dispositif comporte en outre des moyens de recueil de dépolarisations auriculaires droites, et des moyens aptes à évaluer la variation, d'un cycle cardiaque au(x) suivant(s), de l'intervalle atrioventriculaire séparant la dépolarisation auriculaire droite de la dépolarisation ventriculaire et, si la variation ainsi évaluée excède un seuil prédéterminé, réduire d'un pas de variation le délai interauriculaire gauche abrégé.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 illustre de façon schématique la position des différents sites impliqués dans l'activité électrique cyclique, spontanée ou stimulée, du coeur.

La Figure 2 est une série de chronogrammes illustrant différents signaux caractérisant l'activité cardiaque au cours de deux cycles successifs, dans le cas d'une technique *d'overdriving* connue.

La Figure 3 est un diagramme illustrant, dans un exemple de réalisation, le séquencement des détections et des stimulations auriculaires et ventriculaires sur quatre cycles cardiaques successifs, dans le cas d'un dispositif fonctionnant conformément aux enseignements de l'invention.

La Figure 4 est un organigramme décrivant la manière de délivrer la stimulation cardiaque suivant un exemple de réalisation de l'invention.

La Figure 5 est un schéma par blocs illustrant un exemple de réalisation d'un dispositif médical implantable pouvant être utilisé pour mettre en oeuvre différentes caractéristiques présentées dans la présente description.

On va maintenant décrire un exemple de réalisation du dispositif de l'invention.

En ce qui concerne ses aspects logiciels, l'invention peut être mise en oeuvre par une programmation appropriée d'algorithmes de commande exécutés par un microcontrôleur ou processeur numérique de signal d'un stimulateur connu, par exemple de type stimulateur cardiaque, resynchroniseur et/ou défibrillateur, comprenant des moyens d'acquisition d'un signal fourni par des sondes endocavitaires.

L'invention peut notamment être appliquée aux dispositifs implantables tels que ceux des familles *Reply*, *Ovatio* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

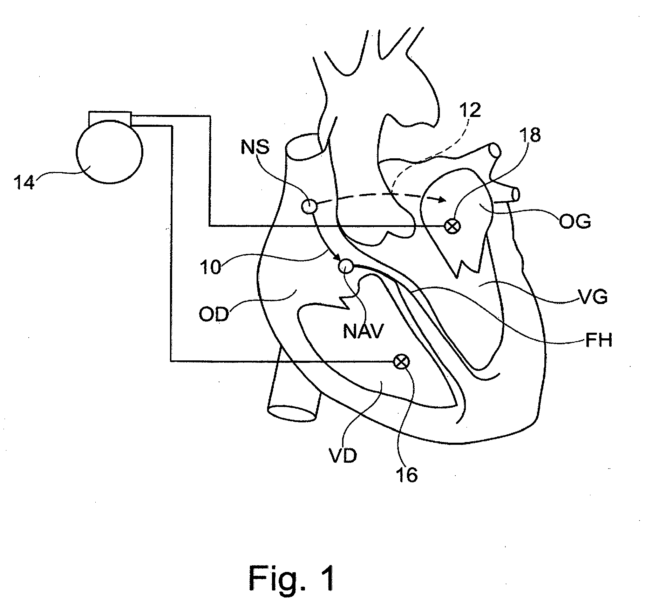

La Figure 1 illustre de façon schématique le coeur avec ses quatre cavités : oreillette droite OD, ventricule droit VD, oreillette gauche OG et ventricule gauche VG.

La contraction coordonnée des différentes cavités prend sa source au noeud sinusal NS, puis l'onde de dépolarisation est conduite au noeud atrioventriculaire NAV (conduction schématisée par la flèche 10), puis à partir de ce noeud au faisceau de His FH, et enfin aux tissus des ventricules droit et gauche VD et VG, provoquant la contraction de ceux-ci.

Par ailleurs, l'onde de dépolarisation issue du noeud sinusal NS provoque la contraction de l'oreillette droite OD puis, après conduction interauriculaire (conduction schématisée par la flèche 12) jusqu'à l'oreillette gauche OG, provoque la contraction de cette dernière.

Dans le cas d'un patient souffrant d'insuffisance cardiaque à fraction d'éjection préservée, la conduction atrioventriculaire (flèche 10, du noeud sinusal NS au noeud atrioventriculaire NAV) est généralement préservée, de même que les voies de conduction permettant d'assurer une contraction synchrone des deux ventricules droit VD et gauche VG.

En revanche, lorsque la conduction interauriculaire (flèche 12) est altérée, cela entraine un retard de la dépolarisation et donc de la contraction de l'oreillette gauche OG par rapport aux ventricules. Ceci induit un mauvais séquencement de la contraction de l'oreillette gauche OG par rapport à celle du ventricule gauche VG, avec une contraction plus ou moins concomitante de ces deux cavités. De ce fait, l'oreillette gauche OG ne peut plus remplir correctement sa fonction, qui est de terminer le remplissage du ventricule gauche VG.

Le dispositif implanté comporte un générateur 14 relié à une électrode de détection (ou de stimulation/détection) ventriculaire 16, par exemple une électrode portée par une sonde endocavitaire logée dans le ventricule droit VD. Il comporte également une électrode 18 de stimulation/détection placée au niveau de l'oreillette gauche OG, par exemple une électrode portée par une sonde positionnée dans le sinus coronaire ou dans une veine tributaire du réseau coronaire telle que la veine de Marshall, ou bien sur le septum interauriculaire, ou encore directement dans l'oreillette gauche après ponction du septum interauriculaire.

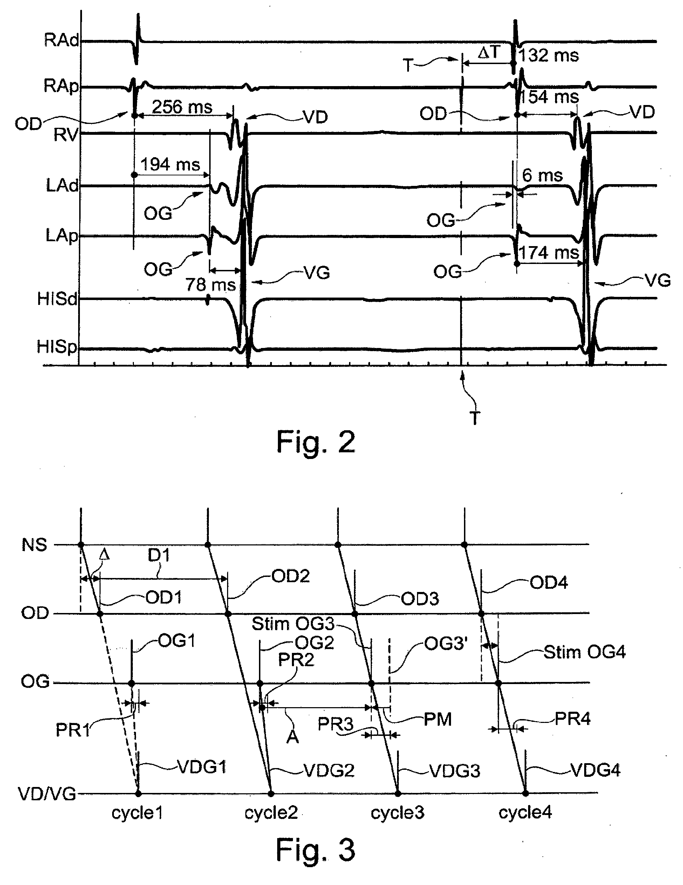

On a illustré sur la Figure 2 la technique conventionnelle *d'overdriving* habituellement mise en oeuvre avec ce type de dispositif pour remédier à une insuffisance à fraction d'éjection préservée.

On a représenté sur cette figure les tracés de divers enregistrements recueillis au cours de deux cycles cardiaques successifs - le premier en rythme spontané, le second avec *overdriving.* Ces enregistrements correspondent aux dérivations suivantes : électrodes distale et proximale sur l'oreillette droite (RAd et RAp), ventricule droit (RV), électrodes distale et proximale sur l'oreillette gauche (LAd et LAp), et électrodes distale et proximale sur le faisceau de His (HISd et HISp), correspondant plus ou moins à la dépolarisation du ventricule gauche (notamment la dérivation HISd).

Au premier cycle cardiaque on constate un retard, conforme, de 256 ms entre la dépolarisation spontanée du ventricule droit (VD) et la dépolarisation de l'oreillette droite (OD). En revanche, le retard OD-OG entre les dépolarisations respectives des deux oreillettes présente une valeur, excessive et pathologique, de 194 ms, ce qui a pour conséquence de ne laisser qu'un intervalle de 78 ms entre la dépolarisation de l'oreillette gauche (OG) et la contraction du ventricule gauche (VG), intervalle trop bref pour permettre un remplissage satisfaisant de ce ventricule gauche. Dans certains cas extrêmes, la contraction de l'oreillette gauche et celle du ventricule gauche peuvent même être quasi-concomitantes, réduisant à néant le rôle de l'oreillette dans le remplissage du ventricule.

Au second cycle cardiaque, qui est un cycle avec *overdriving*, le dispositif stimule l'oreillette gauche à un instant donné (T) présentant une prématurité délibérée $\Delta T$ par rapport au rythme sinusal normal correspondant à l'intervalle de couplage ventriculaire d'un cycle au suivant. Cette stimulation prématurée de l'oreillette gauche permet de rétablir un séquencement OG-VG pratiquement physiologique (174 ms) ; en revanche, le séquencement OD-VD est significativement modifié, puisque ce délai est maintenant de 154 ms au lieu de 256 ms. Lorsque cette réduction est importante, elle peut avoir pour conséquence des effets délétères en ce qui concerne le régime hémodynamique côté droit.

La Figure 3 est un diagramme illustrant le séquencement des détections et des stimulations auriculaires et ventriculaires sur quatre cycles cardiaques successifs, dans le cas d'un dispositif fonctionnant conformément aux enseignements de l'invention.

On a représenté sur ce diagramme divers marqueurs correspondant :

- au rythme sinusal NS,
- au rythme auriculaire droit OD (non accessible à la mesure),
- au rythme auriculaire gauche OG (détecté par l'électrode 18), et
- au rythme ventriculaire VD/VG (détecté par l'électrode 16 ; on fera l'hypothèse qu'il n'y a pas de délai entre la dépolarisation du ventricule droit et celle du ventricule gauche).

Le principe de base de l'invention consiste à commander le dispositif avec une stimulation de l'oreillette gauche contrôlée de manière à éviter tout effet *d'overdriving* et ainsi ne pas modifier la séquence atrioventriculaire droite.

Initialement (Cycle 1), l'influx initial né dans le noeud sinusal se propage dans l'oreillette droite : le marqueur OD1 correspond à une détection qui serait délivrée par une sonde auriculaire droite, avec un retard $\Delta$ par rapport au début du cycle, retard correspondant au délai de propagation jusqu'à l'oreillette droite depuis le noeud sinusal.

Cet influx dépolarise ensuite le noeud atrioventriculaire et atteint les ventricules (marqueur VDG1). Il se propage également vers l'oreillette gauche (marqueur OG1), avec un retard important dans la pathologie d'insuffisance à fraction d'éjection préservée, de sorte que dans l'exemple illustré, la dépolarisation de l'oreillette gauche (marqueur OG1) et celle des ventricules (marqueur VDG1) sont pratiquement concomitantes.

Le cycle se répète (Cycle 2), avec un intervalle de couplage auriculaire D1 correspondant au rythme sinusal. Le défaut de séquencement de l'oreillette gauche se reproduit, avec un intervalle D2 trop long entre les dépolarisations des oreillettes à droite (marqueur OD2) et à gauche (marqueur OG2).

Au cycle suivant (Cycle 3), conformément à l'invention, une stimulation est délivrée à l'oreillette gauche (marqueur StimOG3) après une durée A par rapport à la précédente dépolarisation de l'oreillette gauche (marqueur OG2). Cette durée est choisie pour engendrer une prématurité PM prédéterminée (par rapport au marqueur OG3' de la dépolarisation que l'on aurait eue avec l'intervalle de couplage auriculaire normal), calculée de manière à :

- s'assurer que l'intervalle OG3-VDG3 est suffisamment long pour établir un séquencement normal des cavités gauches, et
- ne pas modifier le séquencement OD3-VDG3 des cavités droites (cette information n'étant pas évaluable par le dispositif sauf si ce dernier est du type "triple chambre", muni d'une sonde auriculaire droite).

Pour ce faire, le dispositif détermine les durées PR1 (OG1-VDG1) et PR2 (OG2-VDG2) au cours des cycles n°1 et 2, et calcule une prématurité telle que l'intervalle interauriculaire AA soit donné par :

$$AA = RR\text{-}PM$$

RR étant l'intervalle de couplage ventriculaire (détecté par l'électrode ventriculaire 16), et
PM étant un intervalle de temps fixe, par exemple de 150 ms, ou programmable.

Au cycle suivant (Cycle 4) le dispositif délivre à l'oreillette gauche une stimulation (marqueur StimOG4), mais avec un intervalle interauriculaire D3 correspondant à l'intervalle de couplage RR qui avait été mesuré au cours des Cycles 1 et 2.

Le dispositif mesure alors l'intervalle PR3 (OG3-VDG3) et compare cet intervalle à l'intervalle PR1 (intervalle mesuré OG1-VDG1 qui peut, en variante, être remplacé par une valeur cible programmable correspondant à la valeur attendue de l'intervalle de couplage en rythme sinusal) :

- si PR3 = PR1+ PM, ceci signifie que la prématurité est suffisante et que la séquence OG-VG est restaurée. Un second contrôle peut être effectué en vérifiant la stabilité de l'intervalle RR3 par rapport à RR1 ;
- si RR3 < RR1, ceci signifie soit que le rythme s'est accéléré, soit que la prématurité était trop importante. Il faut alors refaire le test avec une prématurité PM plus faible (réduite d'un pas, typiquement 8 ms) jusqu'à trouver une séquence n telle que l'intervalle PRn=PR1 ;
- dans le cas où l'on a prévu, comme indiqué plus haut, une valeur cible programmable, si l'intervalle atrioventriculaire n'augmente pas au-delà de cette valeur-cible et que le délai interventriculaire demeure voisin de l'intervalle de couplage du rythme sinusal, il convient alors d'augmenter d'un pas le délai interauriculaire gauche abrégé, puis de délivrer une impulsion de stimulation auriculaire gauche prématurée au cours d'un cycle ultérieur, avec application de ce délai accru d'un pas.

De façon régulière, par exemple toutes les minutes, ou en cas de variation significative de l'intervalle atrioventriculaire PR ou de l'intervalle de couplage ventriculaire RR dépassant un seuil donné, le processus ci-dessus est réitéré, de manière à disposer d'un cycle sans stimulation de l'oreillette gauche, qui servira de référence.

Dans l'hypothèse d'un dispositif "triple chambre" pourvu de moyens de recueil de dépolarisations auriculaires droites, il est possible d'évaluer la variation de l'intervalle atrioventriculaire droit d'un cycle au(x) suivant(s), variation qui révèlerait, au-delà d'un certain seuil, un impact négatif de l'*overdriving* et devrait conduire à réduire la prématurité initialement appliquée.

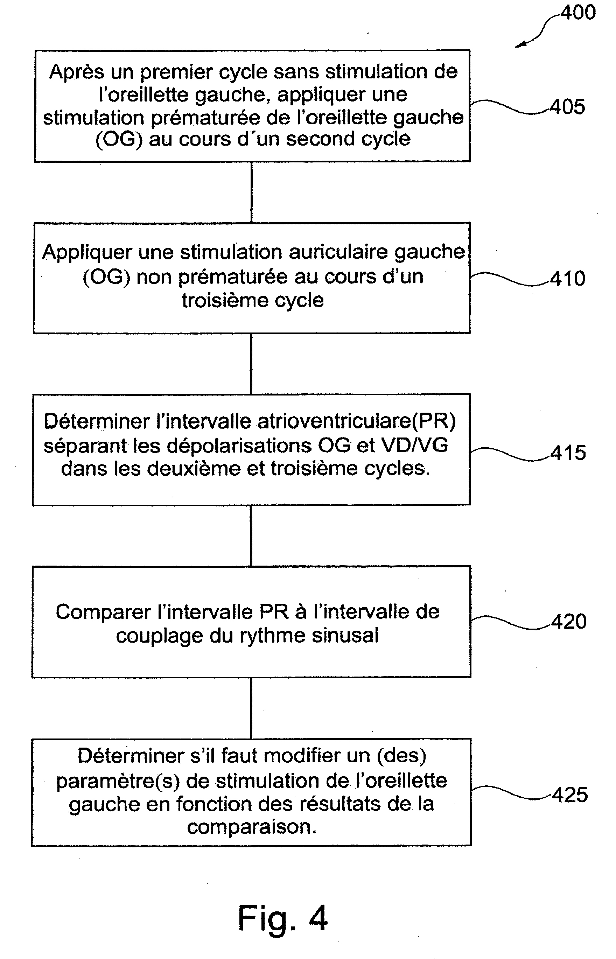

On a représenté sur la Figure 4 un schéma de principe d'un organigramme 400 de délivrance de la stimulation cardiaque selon un exemple de réalisation de l'invention.

Après un premier cycle cardiaque au cours duquel aucune stimulation auriculaire gauche OG n'est délivrée, le dispositif (par exemple, un dispositif médical implantable) délivre une impulsion de stimulation OG prématurée lors du deuxième cycle cardiaque (étape 405), qui suit immédiatement le premier cycle cardiaque.

La stimulation OG prématurée est ajustée de façon à entrainer l'application d'un délai interauriculaire gauche abrégé plus court que l'intervalle de couplage du rythme sinusal.

Le dispositif applique ensuite une impulsion de stimulation OG non prématurée au cours d'un troisième cycle cardiaque (étape 410), qui suit immédiatement le deuxième cycle cardiaque. L'impulsion de stimulation OG non prématurée est ajustée de façon à entrainer l'application d'un délai interauriculaire gauche qui correspond à l'intervalle de couplage de la période sinusale.

Le dispositif détermine ensuite un intervalle atrioventriculaire (intervalle PR) séparant la dépolarisation de l'oreillette gauche de celle des ventricules lors des deuxième et troisième cycles (étape 415). Le dispositif compare l'intervalle atrioventriculaire PR à l'intervalle de couplage du rythme sinusal (étape 420). En fonction du résultat de la comparaison, le dispositif détermine s'il y a lieu et/ou comment modifier un ou plusieurs paramètres de la stimulation de l'oreillette gauche appliquée lors des cycles cardiaques ultérieurs (étape 425). Une ou plusieurs étapes de l'organigramme 400 peuvent être répétées à la suite de la modification des paramètres, pour modifier davantage les paramètres et/ou optimiser la stimulation de l'oreillette gauche.

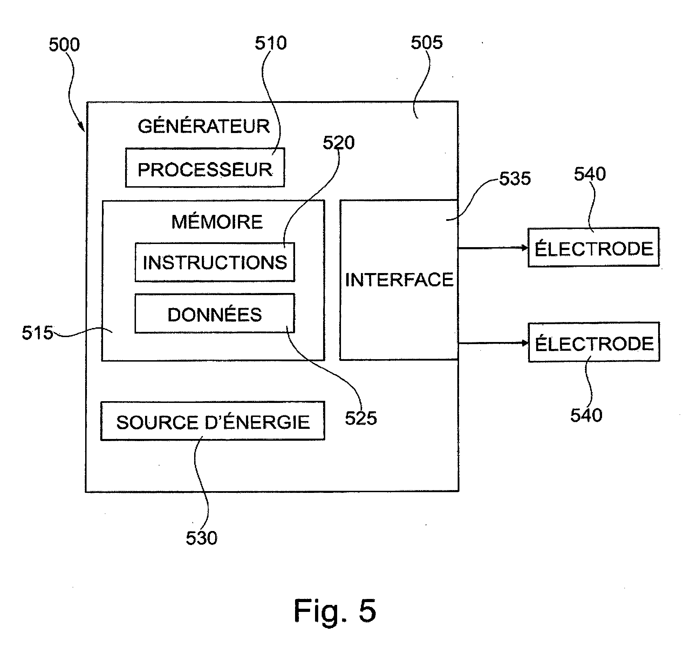

On a représenté sur la Figure 5 un schéma de principe d'un exemple de réalisation d'un dispositif 500 susceptible d'être utilisé pour mettre en oeuvre différentes fonctionnalités décrites ci-dessus.

Le dispositif 500 peut être un dispositif médical implantable conçu pour être implanté chez un sujet, par exemple un patient. Le dispositif 500 comprend un générateur 505 configuré pour générer des signaux de stimulation de tissus, par exemple un tissu cardiaque, un tissu nerveux, etc.). Des signaux de détection peuvent également être reçus en provenance d'une ou plusieurs électrodes 540 couplées au générateur 505 via une interface 535 filaire ou sans fil. Dans certains modes de réalisation, le générateur 505 peut être connecté à des électrodes 540 par des câbles. Également, dans certains modes de réalisation le générateur 505 peut être connecté à des électrodes et inclure une source d'énergie 530 telle qu'une batterie. Dans certains modes de réalisation, le générateur 505 peut comprendre un ou plusieurs émetteurs-récepteurs sans fil configurés pour permettre au générateur 505 de communiquer sans fil avec un ou plusieurs autres dispositifs (par exemple, des dispositifs externes au patient). Par exemple, un émetteur-récepteur sans fil peut communiquer avec un dispositif informatique d'usage général ou à usage spécial (par exemple, un ordinateur de bureau, une tablette, etc.), notamment dans un hôpital ou un environnement clinique pour transmettre des signaux vers et depuis le dispositif informatique externe, par exemple recevoir des signaux de commande configurés pour commander les paramètres de détection/stimulation du générateur 505 et/ou fournir des données relatives à la détection/stimulation au dispositif informatique externe.

Le générateur 505 comprend un processeur 510 et une mémoire 515. Le processeur 510 peut être de n'importe quel type standard ou être un processeur pour usages spéciaux susceptible d'être intégré dans le boitier du générateur 505. La mémoire 515 peut inclure tout type de support approprié de stockage lisible par machine pour stocker des instructions exécutables par machine 520 et/ou d'autres données 525. Les instructions 520 peuvent être exécutées par le processeur 510 pour mettre en oeuvre diverses opérations décrites dans le présent exposé. À titre d'exemple, de tels supports de stockage lisibles par une machine peuvent inclure des mémoires RAM, ROM, EPROM, EEPROM, mémoire flash ou tout autre moyen qui peut être utilisé pour transférer ou stocker du code de programme sous la forme d'instructions exécutables par machine ou de structures de données et qui peut être lu par une machine avec un processeur. Toute combinaison de ce qui précède est également incluse dans le champ d'application de ce que l'on désigne comme étant un support de stockage lisible par machine. Les machines ou supports de stockage lisibles par ordinateur mentionnés ici ne comprennent pas les supports temporaires, par exemple les signaux en champ libre.

Dans certains modes de réalisation, la mémoire 515 peut comporter un ou plusieurs modules avec des instructions configurées pour amener le processeur 510 à réaliser diverses fonctions telles que celles décrites précédemment. Par exemple, la mémoire 515 peut comprendre un module de stimulation configuré pour commander la génération et/ou la transmission d'impulsions de stimulation (par exemple, les impulsions de stimulation auriculaire) à des électrodes 540. La mémoire 515 peut comprendre un module de détection configuré pour détecter les informations collectées par les électrodes 540 et évaluer la réponse à fournir (par exemple, l'évaluation de l'intervalle de couplage auriculaire droit et/ou la comparaison de l'intervalle de couplage auriculaire droit et l'intervalle du rythme sinusal). Le module de détection peut être configuré de manière à modifier un ou plusieurs paramètres de la stimulation en fonction de cette évaluation. Les paramètres peuvent également être stockés dans la mémoire 515.

## Revendications

1. Un dispositif médical implantable actif du type prothèse cardiaque de stimulation, resynchronisation et/ou défibrillation, comprenant :

   - des moyens de recueil de dépolarisations ventriculaires (VD) ;
   - des moyens de recueil de dépolarisations auriculaires gauches (OG) ;
   - des moyens de délivrance d'impulsions de stimulation auriculaire gauche (StimOG) ; et
   - des moyens de contrôle de stimulation auriculaire, aptes à délivrer sélectivement lesdites impulsions de stimulation auriculaires gauches de manière prématurée, par application d'un délai interauriculaire gauche abrégé, plus court que l'intervalle de couplage du rythme sinusal,

   les moyens de contrôle de stimulation auriculaire comprenant :

   - des moyens aptes, après un premier cycle cardiaque sans stimulation auriculaire gauche (Cycle 2) à délivrer une impulsion de stimulation auriculaire gauche prématurée au cours d'un deuxième cycle cardiaque immédiatement consécutif (Cycle 3), avec application d'un délai interauriculaire gauche abrégé (D2), plus court que l'intervalle de couplage du rythme sinusal (D1) ;

   **caractérisé en ce que** les moyens de contrôle de stimulation auriculaire comprennent en outre :

   - des moyens aptes à délivrer une impulsion de stimulation auriculaire gauche non prématurée au cours d'un troisième cycle cardiaque (Cycle 4) immédiatement consécutif audit deuxième cycle cardiaque (Cycle 3), avec application d'un délai interauriculaire gauche (D2) correspondant à l'intervalle de couplage du rythme sinusal ; et
   - des moyens aptes à :

     · évaluer l'intervalle atrioventriculaire séparant la dépolarisation auriculaire gauche de la dépolarisation ventriculaire auxdits deuxième et troisième cycles cardiaques, ou le délai interventriculaire séparant deux dépolarisations ventriculaires successives ;
     · comparer l'intervalle atrioventriculaire ainsi évalué à l'intervalle atrioventriculaire en rythme sinusal, ou le délai interventriculaire ainsi évalué au délai interventriculaire en rythme sinusal ; et
     · modifier, ou non, au moins un paramètre des moyens de contrôle de stimulation auriculaire en fonction du résultat de la comparaison.

2. Le dispositif de la revendication 1, dans lequel ledit paramètre modifié ou non en fonction du résultat de la comparaison est le délai interauriculaire gauche abrégé (D2).

3. Le dispositif de la revendication 2, comprenant en outre des moyens aptes, si l'intervalle atrioventriculaire augmente au-delà d'une valeur-cible donnée, ou si le délai interventriculaire est inférieur à l'intervalle de couplage du rythme sinusal (D1), à :

  · réduire d'un pas de variation ledit délai interauriculaire gauche abrégé ; puis
  · délivrer une impulsion de stimulation auriculaire gauche prématurée au cours d'un cycle cardiaque consécutif, avec application dudit délai interauriculaire gauche abrégé réduit d'un pas.

**4.** Le dispositif de la revendication 3, comprenant en outre des moyens aptes, si l'intervalle atrioventriculaire n'augmente pas au-delà de ladite valeur-cible, et si le délai interventriculaire demeure égal à l'intervalle de couplage du rythme sinusal (D1), à :

  · augmenter d'un pas de variation ledit délai interauriculaire gauche abrégé ; puis
  · délivrer une impulsion de stimulation auriculaire gauche prématurée au cours d'un cycle cardiaque consécutif, avec application dudit délai interauriculaire gauche abrégé accru d'un pas.

**5.** Le dispositif de la revendication 1, comprenant en outre des moyens aptes, sur détection d'un évènement prédéterminé, à :

  · inhiber la délivrance de toute impulsion de stimulation auriculaire gauche au cours d'au moins un cycle cardiaque ;
  · mesurer sur au moins un cycle cardiaque l'intervalle de couplage ventriculaire ; et
  · mémoriser cet intervalle de couplage ventriculaire en tant qu'intervalle de couplage du rythme sinusal pour la détermination desdits délais interauriculaire gauche abrégé et interauriculaire gauche correspondant à l'intervalle de couplage du rythme sinusal, au cours des cycles ultérieurs.

**6.** Le dispositif de la revendication 5, dans lequel ledit évènement prédéterminé est l'expiration d'un intervalle de temps fixe prédéterminé.

**7.** Le dispositif de la revendication 5, dans lequel ledit évènement prédéterminé est la détection du dépassement d'un seuil prédéterminé par la variation du délai atrioventriculaire et/ou de l'intervalle de couplage auriculaire et/ou du délai interventriculaire.

**8.** Le dispositif de la revendication 2, comportant en outre :

  - des moyens de recueil de dépolarisations auriculaires droites ; et
  - des moyens aptes à :

    · évaluer la variation, d'un cycle cardiaque au(x) suivant(s), de l'intervalle atrioventriculaire séparant la dépolarisation auriculaire droite de la dépolarisation ventriculaire ; et
  · si la variation ainsi évaluée excède un seuil prédéterminé, réduire d'un pas de variation le délai interauriculaire gauche abrégé.

**Patentansprüche**

**1.** Aktive implantierbare medizinische Vorrichtung vom Typ Herzprothese zur Stimulation, Resynchronisation und/oder Defibrillation, welche aufweist:

  - Mittel zur Erfassung ventrikulärer Depolarisationen (VD);
  - Mittel zur Erfassung linker aurikulärer Depolarisationen (OG);
  - Mittel zur Zuführung von Impulsen zur linken aurikulären Stimulation (StimOG); und
  - Mittel zur Steuerung der aurikulären Stimulation, die geeignet sind, selektiv die Impulse zur linken aurikulären Stimulation vorzeitig zuzuführen, durch Anwendung einer verkürzten linken interaurikulären Verzögerung, die kürzer als das Kopplungsintervall des Sinusrhythmus ist;

wobei die Mittel zur Steuerung der aurikulären Stimulation aufweisen:

  - Mittel, die geeignet sind, nach einem ersten Herzzyklus ohne linke aurikuläre Stimulation (Zyklus 2) einen Impuls zur vorzeitigen linken aurikulären Stimulation während eines zweiten, unmittelbar nachfolgenden Herzzyklus (Zyklus 3) zuzuführen, mit Anwendung einer verkürzten linken interaurikulären Verzögerung (D2), die kürzer als das Kopplungsintervall des Sinusrhythmus (D1) ist;

**dadurch gekennzeichnet, dass** die Mittel zur Steuerung der aurikulären Stimulation außerdem aufweisen:

  - Mittel, die geeignet sind, einen Impuls zur nicht vorzeitigen linken aurikulären Stimulation während eines dritten Herzzyklus (Zyklus 4), der unmittelbar auf den zweiten Herzzyklus (Zyklus 3) folgt, zuzuführen, mit Anwendung einer linken interaurikulären Verzögerung (D2), die dem Kopplungsintervall des Sinusrhythmus entspricht; und
  - Mittel, die geeignet sind:

    • das atrioventrikuläre Intervall, das die linke aurikuläre Depolarisation von der ventrikulären Depolarisation beim zweiten und dritten Herzzyklus trennt, oder die interventrikuläre Verzögerung, die zwei aufeinander folgende ventrikuläre Depolarisationen trennt, zu bestimmen;
• das so bestimmte atrioventrikuläre Intervall mit dem atrioventrikulären Intervall beim Sinusrhythmus oder die so bestimmte interventrikuläre Verzögerung mit der interventrikulären Verzögerung beim Sinusrhythmus zu vergleichen; und
• wenigstens einen Parameter der Mittel zur Steuerung der aurikulären Stimulation in Abhängigkeit vom Ergebnis des Vergleichs zu ändern oder nicht.

2. Vorrichtung nach Anspruch 1, wobei der Parameter, der in Abhängigkeit vom Ergebnis des Vergleichs geändert wird oder nicht, die verkürzte linke interaurikuläre Verzögerung (D2) ist.

3. Vorrichtung nach Anspruch 2, welche außerdem Mittel aufweist, die, falls sich das atrioventrikuläre Intervall über einen gegebenen Zielwert hinaus vergrößert, oder falls die interventrikuläre Verzögerung kleiner als das Kopplungsintervall des Sinusrhythmus (D1) ist, geeignet sind:

    • die verkürzte linke interaurikuläre Verzögerung um einen Änderungsschritt zu verringern; und danach
    • einen Impuls zur vorzeitigen linken aurikulären Stimulation während eines nachfolgenden Herzzyklus zuzuführen, mit Anwendung der um einen Schritt verringerten verkürzten linken interaurikulären Verzögerung.

4. Vorrichtung nach Anspruch 3, welche außerdem Mittel aufweist, die, falls sich das atrioventrikuläre Intervall nicht über den Zielwert hinaus vergrößert, und falls die interventrikuläre Verzögerung gleich dem Kopplungsintervall des Sinusrhythmus (D1) bleibt, geeignet sind:

    • die verkürzte linke interaurikuläre Verzögerung um einen Änderungsschritt zu vergrößern; und danach
    • einen Impuls zur vorzeitigen linken aurikulären Stimulation während eines nachfolgenden Herzzyklus zuzuführen, mit Anwendung der um einen Schritt vergrößerten verkürzten linken interaurikulären Verzögerung.

5. Vorrichtung nach Anspruch 1, welche außerdem Mittel aufweist, die geeignet sind, bei Erkennung eines vorbestimmten Ereignisses:

    • die Zuführung jedes Impulses zur linken aurikulären Stimulation während wenigstens eines Herzzyklus zu verhindern;

    • bei wenigstens einem Herzzyklus das ventrikuläre Kopplungsintervall zu messen; und
    • dieses ventrikuläre Kopplungsintervall als Kopplungsintervall des Sinusrhythmus für die Bestimmung der verkürzten linken interaurikulären und der linken interaurikulären Verzögerung zu speichern, das dem Kopplungsintervall des Sinusrhythmus während der späteren Zyklen entspricht.

6. Vorrichtung nach Anspruch 5, wobei das vorbestimmte Ereignis der Ablauf eines vorbestimmten festen Zeitintervalls ist.

7. Vorrichtung nach Anspruch 5, wobei das vorbestimmte Ereignis die Erkennung der Überschreitung eines vorbestimmten Schwellenwertes durch die Änderung der atrioventrikulären Verzögerung und/oder des aurikulären Kopplungsintervalls und/oder der interventrikulären Verzögerung ist.

8. Vorrichtung nach Anspruch 2, welche außerdem aufweist:

    - Mittel zur Erfassung rechter aurikulärer Depolarisationen; und
    - Mittel, die geeignet sind:

        • die Änderung des atrioventrikulären Intervalls, das die rechte aurikuläre Depolarisation von der ventrikulären Depolarisation trennt, von einem Herzzyklus zum (zu den) folgenden zu bestimmen; und
        • falls die so bestimmte Änderung einen vorbestimmten Schwellenwert überschreitet, die verkürzte linke interaurikuläre Verzögerung um einen Änderungsschritt zu verringern.

## Claims

1. An active implantable medical device such as a cardiac prosthesis for stimulation, resynchronization and/or defribrillation, comprising:

    - means for collecting ventricular depolarizations (VD);
    - means for collecting left atrial depolarizations (OG);
    - means for delivering left atrial stimulation pulses (StimOG); and
    - means for controlling the atrial stimulation, adapted to prematurely and selectively deliver said left atrial stimulation pulses, by applying an abridged left interatrial delay, that is shorter than the sinusal rhythm coupling interval,

the atrial stimulation control means comprising:

- means adapted to deliver, after a first cardiac cycle with no left atrial stimulation (Cycle 2), a premature left atrial stimulation pulse during a second, immediately consecutive cardiac cycle (Cycle 3), with application of an abridged left interatrial delay (D2), that is shorter than the sinusal rhythm coupling interval (D1);

**characterized in that** the atrial stimulation control means further comprise:

- means adapted to deliver a non-premature left atrial stimulation pulse during a third cardiac cycle (Cycle 4) that is immediately consecutive to said second cardiac cycle (Cycle 3), with application of a left interatrial delay (D2) corresponding to the sinusal rhythm coupling interval; and
- means adapted to:

  • evaluate the atrioventricular interval separating the left atrial depolarization from the ventricular depolarization at said second and third cardiac cycles, or the interventricular delay separating two successive ventricular depolarizations;
  • comparing the so-evaluated atrioventricular interval to the atrioventricular interval in sinusal rhythm, or the so-evaluated interventricular delay to the interventricular delay sinusal rhythm ; and
  • modifying, or not, at least one parameter of the atrial stimulation control means as a function of the comparison result.

2. The device of claim 1, wherein said parameter, modified or not as a function of the comparison result is the abridged left interatrial delay (D2).

3. The device of claim 2, further comprising means adapted, if the atrioventricular interval increases beyond a given target-value, or if the interventricular delay is lower than the sinusal rhythm coupling interval (D1), to:

  • reduce by one variation step said abridged left interatrial delay; then
  • deliver a premature left atrial stimulation pulse during a consecutive cardiac cycle, with application of said abridged left interatrial delay reduced by one step.

4. The device of claim 3, further comprising means adapted, if the atrioventricular interval does not increase beyond said target-value, and if the interventricular delay remains equal to the sinusal rhythm coupling interval (D1), to:

  • increase by one variation step said abridged left interatrial delay; then
  • deliver a premature left atrial stimulation pulse during a consecutive cardiac cycle, with application of said abridged left interatrial delay increased by one step.

5. The device of claim 1, further comprising means adapted, upon detection of a predetermined event, to:

  • inhibit the delivery of any left atrial stimulation pulse during at least one cardiac cycle;
  • measure over at least one cardiac cycle the ventricular coupling interval; and
  • memorize this ventricular coupling interval as the sinusal rhythm coupling interval to determine said abridged left interatrial and left interatrial delays corresponding to the sinusal rhythm coupling interval, during the later cycles.

6. The device of claim 5, wherein said predetermined event is the expiry of a predetermined fixed time interval.

7. The device of claim 5, wherein said predetermined event is the detection of the exceeding of a predetermined threshold by the variation of the atrioventricular delay and/or of the atrial coupling interval and/or of the interventricular delay.

8. The device of claim 2, further comprising:

  - means for collecting right atrial depolarizations; and
  - means adapted to:

    • evaluate the variation, from one cardiac cycle to the following one(s), of the atrioventricular interval separating the right atrial depolarization from the ventricular depolarization; and
    • reduce by one variation step the abridged left interatrial delay, if the so-evaluated variation exceeds a predetermined threshold.

Fig. 1

Fig. 2

Fig. 3

_/400_

```
Après un premier cycle sans stimulation de
l'oreillette gauche, appliquer une
stimulation prématurée de l'oreillette gauche      ___405
(OG) au cours d'un second cycle
```

```
Appliquer une stimulation auriculaire gauche
(OG) non prématurée au cours d'un                  ___410
troisième cycle
```

```
Déterminer l'intervalle atrioventriculare(PR)
séparant les dépolarisations OG et VD/VG           ___415
dans les deuxième et troisième cycles.
```

```
Comparer l'intervalle PR à l'intervalle de         ___420
couplage du rythme sinusal
```

```
Déterminer s'il faut modifier un (des)
paramètre(s) de stimulation de l'oreillette        ___425
gauche en fonction des résultats de la
comparaison.
```

# Fig. 4

500   510   505

GÉNÉRATEUR

PROCESSEUR   520

535   540

MÉMOIRE

INSTRUCTIONS   ÉLECTRODE

INTERFACE

DONNÉES

515   ÉLECTRODE

SOURCE D'ÉNERGIE   525   540

530

Fig. 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2471575 A1

- US 8583234 B1